# EUROPÄISCHE PATENTSCHRIFT

(11) **EP 0 590 341 B1**
(45) Veröffentlichungstag und Bekanntmachung des Hinweises auf die Patenterteilung: **12.01.2000**
(21) Anmeldenummer: 93114076.8
(22) Anmeldetag: 02.09.1993
(51) Int. Cl.: C12M 1/12, C12M 3/06, A61F 2/02

(54) **Modul zur Züchtung und zur Nutzung der Stoffwechselleistung und/oder zum Erhalt von Mikroorganismen**
Module for the culture and the use of the metabolic activity or the obtention of microorganisms
Module pour la culture et l'utilisation du rendement métabolique ou l'obtention de micro-organismes

(30) Priorität: 09.09.1992 DE 4230194
(43) Veröffentlichungstag der Anmeldung: 06.04.1994
(73) Patentinhaber: Gerlach, Jörg, Dr., D-10965 Berlin (DE)
(72) Erfinder: Gerlach, Jörg, Dr., D-10965 Berlin (DE)
(74) Vertreter: Pfenning, Meinig & Partner

(56) Entgegenhaltungen:
- EP-A- 0 113 328
- EP-A- 0 419 234
- WO-A-86/06094
- WO-A-91/15570
- US-A- 5 081 035

## Beschreibung

Die Erfindung betrifft ein Modul zur Züchtung und zur Nutzung von Stoffwechselleistungen und/oder zum Erhalt von Mikroorganinsmen, insbesondere von Zellen oder Bakterien sowie ein Verfahren zum Betreiben des Moduls und die Verwendung des Moduls.

Vorrichtungen für den Stoffaustausch, z.B. Bioreaktoren, Zellperfusionseinrichtungen oder allgemein Module, insbesondere im Bereich von Leberunterstützungssystemen, als alternative Methode für Tierversuche oder zur Herstellung von biologischen Zellprodukten, sind bekannt.

In der DE-OS 38 37 226 wird ein Perfusionsgerät zum Züchten und Erhalt von Hepatozyten beschrieben. Dieses Perfusionsgerät ist dadurch gekennzeichnet, daß es eine Kammer mit Zellkompartment und Perfusionskompartment, welche durch eine semipermeable Membran getrennt sind, enthält. Die Hepatozyten sind dabei an den Träger im Hepatozyten-Kompartment immobilisiert. Zusätzliche Hohlfasermembrane im Zellkompartment enthalten Galle.

Die EP 0 419 234 A2 beschreibt eine Zellkulturvorrichtung mit drei unabhängigen Membransystemen.

Aus der WO 86/06094 ist eine Membranfilteranordnung beschrieben, die aus unabhängigen Membransystemen aufgebaut ist.

Aus der US 3,734,351 ist eine Methode zur leberspezifischen Behandlung von Blut und eine entsprechende Vorrichtung beschrieben. Diese Vorrichtung weist eine Kammer mit drei unabhängigen, übereinandergeschichteten Kompartments, welche durch zwei Flachmembraneinlagen in der Kammer gebildet werden, auf. Das erste Kompartment enthält Blut, dessen Bestandteile durch die erste Membrananlage hindurch in das zweite Kompartment gelangen können. Im zweiten Kompartment befinden sich Leberzellen, welche die Blutbestandteile verstoffwechseln. Eine zweite Membran teilt dieses Zellkompartment von einem dritten Kompartment, in welchem sich eine Dialysatflüssigkeit befindet. In dieses dritte Kompartment diffundieren Stoffe aus dem Zellkompartment, welche hieraus abtransportiert werden können.

Der Stoffaustausch zwischen den Zellen und dem Blut oder Mediumkompartment entsteht bei diesem Stand der Technik durch Diffusion. Dies wirkt sich aber nachteilig auf den Zellstoffwechsel und dessen Steuerbarkeit aus. Bedingt durch die bauliche Ausgestaltung, nämlich Bauformen, die dadurch gekennzeichnet sind, daß das Medium auf einer Seite einströmt und nach der Diffusion durch die Membrane wieder austritt, ergibt sich zwischen dem Mediumeinfluß und dem Mediumausfluß ein unterschiedlicher Beitrag für den Zellstoffaustausch. Im Anfangsbereich findet ein erhöhter Stoffaustausch statt, der dann im Laufe des Hindurchfließens des Mediums durch die Hohlfasermemebrane abnimmt. Dies hat zur Folge, daß die Zellen einen unterschiedlichen Beitrag bezüglich des Stoffaustausches ausgesetzt sind. All dies führt dazu, daß kein gleichmäßiger Stoffaustausch stattfindet und z.B. der Metabolismus von Zellen und deren Lebensfähigkeit deutlich beeinträchtigt wird.

Aus der FR 2 660 323 ist ebenfalls ein Modul zur Züchtung von Zellen bekannt, welches drei unabhängige Membransysteme enthält. Die unabhängigen Membransysteme sind aber auch hier zueinander so beabstandet, daß hinsichtlich der Ver- und Entsorgung erhebliche Stoffgradienten auftreten.

Hier setzt die vorliegende Erfindung ein, deren Aufgabe es ist, ein Modul für den Stoffaustausch zwischen Mikroorganismen, insbesondere Zellen, und einem Medium anzugeben, das es ermöglicht, daß der Stoffaustausch und die Steuerbarkeit des Stoffaustausches verbessert werden und daß z.B. der Metabolismus und die Lebensfähigkeit von Zellen verbessert werden.

Die Aufgabe wird durch den Anspruch 1 gelöst. Die Unteransprüche 2 bis 7 betreffen vorteilhafte Weiterbildungen des Moduls.

Das Verfahren zum Betreiben des Moduls ist durch den Anspruch 8 gekennzeichnet. Die Ansprüche 9 bis 15 betreffen Weiterbildungen des Verfahrens.

Besondere Verwendungen des Moduls sind durch die Ansprüche 16 bis 18 gekennzeichnet.

Erfindungsgemäß wird unter Modul eine Stoffaustauschvorrichtung in Form eines dreidimensionalen Körpers verstanden, der aus einem Außengehäuse und einem inneren Bereich besteht, wobei im Inneren des Moduls ein dicht gepacktes räumliches Netzwerk angeordnet ist. Die Form des Moduls kann dabei vielgestaltig sein. Das Modul kann z.B. einen rechteckigen, oder n-eckigen Grundriß aufweisen. Auch kugelförmige oder scheibenförmige Module sind für die erfindungsgemäße Lehre geeignet.

Erfindungswesentlich ist nun, daß mindestens drei unabhängige Memebransysteme eingesetzt werden, wobei diese unabhängigen Membransysteme als Hohlfasermembransysteme ausgebildet sind und daß diese Hohlfassermemebransysteme im Innenbereich ein dicht gepacktes Netzwerk bilden.

Ein erstes unabhängiges Hohlfasermembransystem dient dabei für den Mediumzufluß. Ein zweites unabhängiges Hohlfasermembran ist für die Versorgung der Mikroorganismen, z.B. mit Sauerstoff bzw. die Entsorgung mit CO₂, vorgesehen. Der Mediumabfluß wird durch ein drittes unabhängiges Membransystem sichergestellt.

Jedes einzelne unabhängige Hohlfasermembransystem besteht dabei aus einer Vielzahl einzelner Hohlfasermembranen, wobei jeweils die Hohlfasern eines Systems mit mindestens einem Einlaß bzw. Einlaß und einem Auslaß kommunizieren. Damit wird sichergestellt, daß die Hohlfasern eines jeweiligen unabhängigen Systems gleichzeitig durch den Einlaß z.B. mit Medium versorgt werden können.

Die unabhängigen Hohlfasermembransysteme bilden nun im Innenbereich des Moduls ein räumlich dicht gepacktes Netzwerk und zwar in der Weise, daß nahezu an jeder Stelle des Netzwerkes wenige Mikroorganismen nahezu identische Bedingungen für die Substratversorgung haben. Dadurch sind die Verhältnisse in physiologischen Organen mit eigenen Arterien und Venen, wie z.B. der Leber, mit der Anordnugn von Hepatozyten in Lobuli weitgehend simuliert. Durch die unabhängige Anordnung der verschiedenen Membransysteme bietet das Modul den Vorteil eines dezentralen Transportes von z.B. Nährstoffen, Syntheseprodukten und Gasen zu- und von einer Vielzahl von Mikroorganinsmen unabhängig von ihrer Position im Modul, wie es in der Zellumgebung in natürlichen Organen der Fall ist.

Das dicht gepackte Netzwerk kann dabei verschieden aufgebaut sein, solange nur gewährleistet ist, daß jeweils wenige Mikroorganismen im Innenbereich, eine identische Substratversorgung haben. Das räumliche dicht gepackte Netzwerk kann z.B. aus dicht gepackten Schichten bestehen, wobei sich jeweils Schichten vom unabhängigen System abwechseln. Eine erste Schicht, die aus einzelnen Hohlfasermembranen besteht, ist dabei horizontal angeordnet. Die zweite Schicht, die jeweils wieder aus einzelnen Hohlfasermembranen besteht, ist dabei ebenfalls in der gleichen Ebene aber gegenüber der ersten Schicht verdreht z.B. in einem Winkel von 90° angeordnet. Diese Schichten wechseln sich nun ab und bilden zusammen eine dichte Packung. Das dritte unabhängige Hohlfasermembransystem, das jeweils wiederum aus einzelnen Schichten von Hohlfasermembranen besteht, durchzieht nun diese beiden Schichten z.B. vertikal von oben nach unten und "verwebt" somit die beiden ersten unabhängien Schichten miteinander.

Eine weitere Ausführung sieht vor, drei unabhängige Hohlfasermembransysteme in sich abwechselnden Schichten so übereinander zu legen, daß sie alle in einer Ebene liegen, aber jeweils um z.B. 60° verdreht angeordnet sind.

Dieses dicht gepackte Netzwerk ist nun im Innenbereich des Moduls angeordnet. Dadurch, daß jedes unabhängige System mit mindestens einem Einlaß bzw. einem Einlaß und einem Auslaß kommuniziert, ist nun gewährleistet, daß das zuführende Medium an alle Orte im Modul gleichförmig geführt wird, ebenso wie eine gleichförmige Sauerstoffzufuhr erreicht wird. Durch das dritte unabhängige System für den Mediumabfluß kann nun auch kontinuierlich und gleichmäßig das Medium aus dem gesamten Modul, an jeder Stelle, abgeführt werden.

In einer bevorzugten Ausführungsform wird vorgeschlagen, daß zusätzlich zu den drei Hohlfasermembransystemen im Innenbereich ein weiteres unabhängiges Membransystem für den Mediumabfluß verwendet wird. Erfindungsgemäß kann dazu am Außengehäuse einweder eine auswechselbare Flachmembran oder eine auswechselbare Kapillarmembran angebracht werden. Durch diese erfindungsgemäße Ausgestaltung ist sichergestellt, daß ein problemloser Abfluß des Mediums, auch über längere Zeit, erreicht werden kann.

Erfindungsgemäß werden als Hohlfasermembrane bevorzugt Polypropylen, Polyamid, Polysulphon oder Cellulose oder Silikon verwendet. Die Auswahl der Hohlfasermembrane richtet sich dabei nach den Molekülen, die für den Stoffaustausch vorgesehen sind. Erfindungsgemäß lassen sich aber alle gängigen Hohlfasermembrane, die bereits aus dem Stand der Technik für Stofaustauschvorrichtungen bekannt sind, anwenden.

Erfindungsgemäß wird bei Verwendung von drei unabhängigen Hohlfasermembransystemen, die im Innenbereich ein dicht gepacktes Netzwerk bilden, ein Kapillarsystem aus flüssigkeitsimpermeablen Kapillaren, wie z.B. aus Edelstahl oder Glas verwendet. Dieses kann dann der Temperierung des Moduls mit seinem Innenraum dienen. Ebenso ermöglicht es ein gleichförmiges Abkühlen des Moduls mit seinem Innenraum und eingebrachten Mikroorganismen unter -20 °C. In einer weiteren erfindungsgemäßen Ausgestaltung können aber auch alle weiteren Hohlfasersysteme zur Temperierung bzw. zum Abkühlen bis unter den Gefrierpunkt verwendet werden.

In einer weiteren bevorzugten Ausgestaltung wird vorgeschlagen, daß das Außengehäuse durch eine Ausgußmasse gebildet wird, wobei jeweils immer sichergestellt wird, daß ein Zugang von außen in das Volumen der Kapillaren ermöglicht wird.

Das Modul weist in einer weiteren Ausgestaltung noch verschiedene Zugänge auf. Ein erster Zugang dient dabei dazu, um die Mikroorganismen in das Modul einzufüllen. Weitere Zugänge dienen zur Druck-, pH- und Temperaturmessung im Innenbereich des Moduls.

Die Erfindung betrifft weiterhin ein Verfahren zum Betreiben des Moduls. Erfindungsgemäß wird dabei der Innenbereich des Moduls mit Mikroorganismen gefüllt. Im Anschluß daran werden die für die Züchtung und/oder Erhalt nötigen physiologischen Bedingungen eingestellt. In ein erstes unabhängies Hohlfasermembransystem wird dann das Medium zugeführt. Das Medium tritt durch die Hohlfasermembran hindurch und verstoffwechselt sich mit den Mikroorganismen, die sich entweder in den Hohlräumen befinden oder an den Membranen adhäriert sind. Der Stoffaustausch zwischen den Mikroorganismen und dem Medium erfolgt dabei durch Konvektion. Um dies zu gewährleisten, muß lediglich der Ausgang, an dem unabhängigen Hohlfasermembransystem, in dem das Medium zugeführt wird, verschlossen werden, so daß das Medium durch die Hohlfasermembrane hindurchtreten muß. Das erfindungsgemäße Modul kann aber auch im Diffusionsbetrieb betrieben werden, indem nämlich der Ausgang beim unabhängigen Hohlfasermembransystem für den Mediumfluß offengelassen wird und somit nur ein Diffusionsbetrieb stattfindet. Der besondere Vorzug der Erfindung ist aber darin zu sehen, daß durch den Konvektionsbetrieb ein optimaler und steuerbarer Stoffaustausch erfolgt, wohingegen beim Stand der Technik bisher lediglich ein Stoffaustausch durch Diffusion stattfindet.

Die Erfindung bietet weiterhin den Vorteil, daß die Hohlfasermembranen bzw. Kapillaren mit einem für die Zellen bzw. Mikroorganismen geeignetem Substrat zur Vermittlung der Adhäsion der Mikroorganismen an die Kapillarsysteme beschichtet werden können.

Ein weiterer Vorzug der Erfindung ist, daß zusätzlich zum verwendeten Zelltyp ein oder mehrere weitere Zelltypen in Kokulturtechnik in den Innenraum eingebracht werden können. Diese zusätzliche Zelltypen können dabei in dem Hohlraum eines oder mehrerer unabhängier Kapillarsysteme kultiviert werden. Zellen für die Kokultivierung sind z.B. Nichtparenchymzellen aus den Lebersinusoiden.

Das erfindungsgemäße Modul bietet weiterhin den Vorteil, daß eines der unabhängigen Membransysteme zum Abtransport z.B. von Bestandteilen der Lebergalle dienen kann.

Eine weitere vorteilhafte Variante sieht vor, daß eines der unabhängigen Membransysteme zur Dialyse zu verwenden ist.

Durch die vorgeschlagenen unabhängigen Membransysteme ist somit eine große Breite und Anwendungsmöglichkeiten des Moduls gegeben.

Eine besonders bevorzugte Ausführungsform sieht vor, daß die Mikroorganismen Zellen und hier insbesondere Leberzellen sind.

Weitere Merkmale, Einzelheiten und Vorzüge der Erfindung ergeben sich aus den Figurenbeschreibungen 1 bis 10. Hierbei zeigen:
- Fig. 1: Querschnitt durch ein Modul mit rechteckiger Bauform,
- Fig. 2: jeweils einzelne Hohlfasermembrane, entsprechend Fig. 1, von drei unabhängigen Membransystemen,
- Fig. 3: unabhängige Hohlfasermembransysteme entsprechend Fig. 1 mit Zellkulturen,
- Fig. 4: jeweils einzelne Kapillare von vier unabhängigen Systemen
- Fig. 5: dreidimensionale Darstellung einer Ebene eines Moduls mit vier unabhängigen Hohlfasermembransystemen, entsprechend Fig. 4,
- Fig. 6: drei Möglichkeiten des Mediumausflusses aus dem Modul,
- Fig. 7: alternative Anordnungen der Membranköpfe auf einer Seite des Moduls,
- Fig. 8: Querschnitt eines Moduls mit Hohlfasermembranen und Membranköpfen mit verschieden ausgestalteten Hohlfasermembranen,
- Fig. 9: verschiedene Zugänge zum Modul und
- Fig. 10: exemplarische Verwendung eines Moduls als extrakorporales Unterstützungssystem.

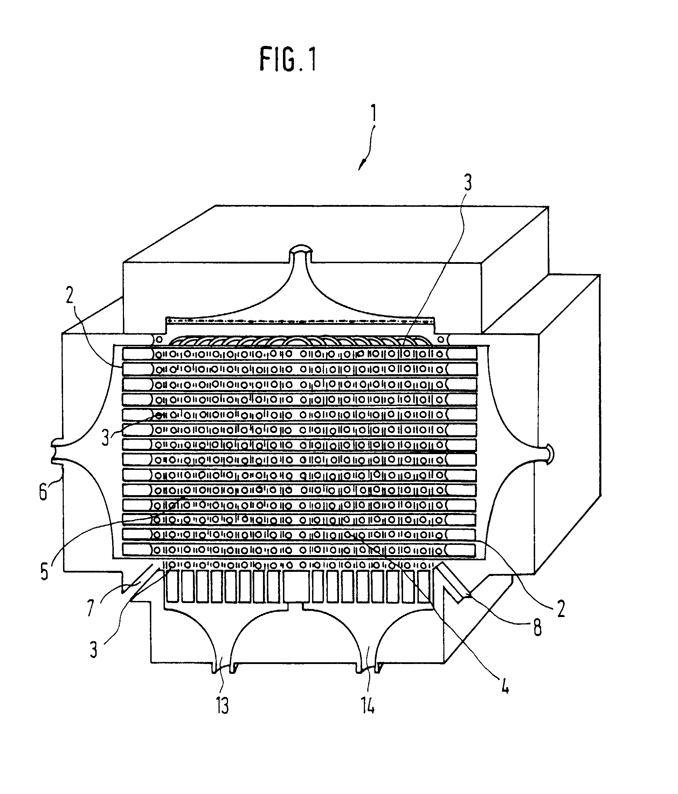

Fig. 1 zeigt einen vertikalen Querschnitt durch eine mögliche Ausgestaltung eines Moduls. Dieses Modul 1 weist ein dicht gepacktes Netzwerk 5 aus drei unabhängigen Systemen auf.

Das Modul 1 besteht aus einem Außengehäuse 2 und einem Innenbereich 4, in dem sich das dicht gepackte Netzwerk 5 befindet. Das Außengehäuse 2 besteht aus einer Dichtmasse, in die die Kapillare vergossen sind. Das Außengehäuse 2 weist dabei einen Zugang von außen in das Lumen der Kapillaren auf. Die Dichtmasse erhält die mechanische Stabilität des Moduls.

Das dicht gepackte Netzwerk 5, im Innenbereich 4 des Moduls 1, ist dabei im Beispielsfall wie folgt aufgebaut:

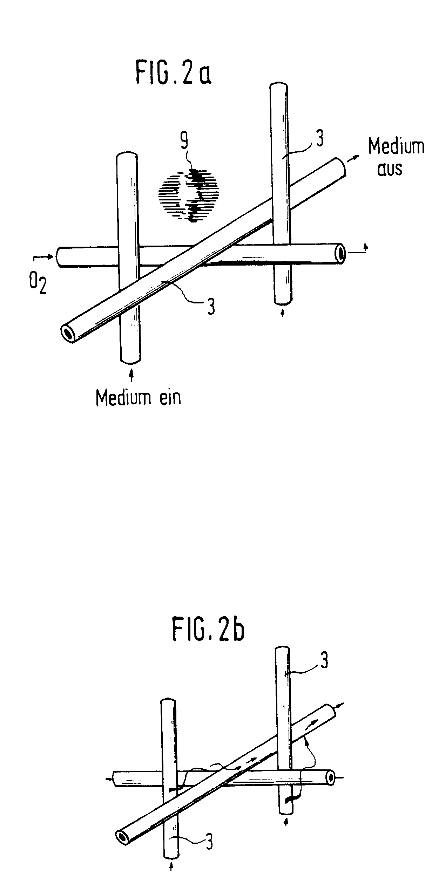

Ein erstes unabhängiges Hohlfasermembransystem wird durch Schichten von Hohlfasern 3 gebildet, wobei diese ersten Schichten horizontal angeordnet sind. Jede einzelne Schicht besteht dabei aus linearen Hohlfasermembranen 3, die dicht nebeneinander angeordnet sind. Das zweite unabhängige Hohlfasermembransystem wird dabei ebenfalls durch Schichten gebildet, die in diesem Fall in einen Winkel von 90° in derselben Ebene angeordnet sind, wobei sich die Schichten der beiden unabhängigen Systeme abwechseln. Diese Schichten sind in Fig. 1 zum Teil durch Kreise dargestellt. In einem Modul 1 sind nun diese abwechselnden Schichten dicht übereinander gepackt angeordnet. In einem Versuchsmodul mit den Außenabmessungen 12 x 12 cm sind dabei ca. 100 Schichten übereinander angeordnet. Das dritte unabhängige Hohlfasermembransystem ist nun im Beispielsfall nach Fig. 1 durch die vertikalen Linien dargestellt. Diese Linien stellen Hohlfasermembrane dar, die nun die in einer Ebene liegenden Schichten vertikal von oben nach unten durchziehen. In dem beschriebenen Versuchsmodul sind dabei ca. 50 Schichten vorgesehen, die das Modul vertikal von oben nach unten verweben. Durch diese Ausgestaltung des dicht gepackten Netzwerkes ist es nun gewährleistet, daß wenige Zellen, wie in Fig. 2a und 2b ersichtlich, jeweils eine identische Substratversorgung haben.

Zum Betrieb des Moduls wird nun z.B. Medium durch den Mediumeinstromkopf 6 in das Lumen der Kapillaren zugeführt. Der dem Mediumeinstromkopf 6 gegenüberliegende ist geschlossen. Dieses Modul tritt nun durch die Membrane der Kapillare hindurch und es tritt ein Stoffaustausch mit den Zellen 9, die entweder in den Hohlräumen angeordnet sind und/oder an den Kapillaren adhäriert sind, ein. Die Zellen 9 werden dann während des Stoffaustausches mit Sauerstoff versorgt. Der Sauerstoff tritt dabei durch den Sauerstoffeinstromkopf 13 in das Lumen der Kapillare ein und wird über den Sauerstoffausstromkopf 14 abgeführt. Im Beispielsfall sind die Hohlfasermembrane 3 des unabhängigen Systems, für die Sauerstoffversorgung, durch U-förmige Hohlfasermembrane gebildet. Der Stoffabtransport erfolgt dann über das dritte unabhängige Hohlfasermembransystem durch die mit Kreisen gekennzeichneten Hohlfasermembranen. Diese Hohlfasermembrane münden dann in einen Mediumausstromkopf 15, von wo aus das Medium abgeführt werden kann (nicht abgebildet).

Durch den Zugang 7 können in dem Innenbereich des Moduls 1 Zellen 9 zugeführt werden. Ein weiterer Zugang 8 ist vorgesehen, um Messungen im Innenbereich des Moduls wie z.B. Druck, Temperatur oder pH vornehmen zu können.

Die gezeigte mögliche Ausgestaltung des Moduls kann beliebig variiert werden. Die Grundform des Moduls kann nicht nur rechteckig sein, sondern sie kann genau so ein N-Eck sein oder ein sonstiger beliebiger Hohlkörper, sofern der Hohlkörper es ermöglicht, daß in einem Innenbereich 4 ein dicht verpacktes Netzwerk 5 von unabhängigen Hohlfasermembransystemen untergebracht werden kann. Erfindungswesentlich ist dabei immer die Ausgestaltung des dicht gepackten Netzwerkes 5 des Hohlfasermembransystemes. Abweichend von der in Fig. 1 dargestellten Möglichkeiten ist es genauso möglich, einzelne Schichten dicht gepackt übereinander anzuordnen, wobei jede einzelne Schicht aus abwechselnden unabhängigen Hohlfasermembranen gebildet ist. Eine weitere Möglichkeit besteht darin, die beiden ersten Schichten nicht im Winkel von 90°, sondern in einen Winkel a von 0 bis 180° anzuordnen.

Auch für das dritte unabhängige Holfasermembransystem bestehen beliebige Möglichkeiten, solange nur gewährleistet ist, daß eine nahezu identische Substratversorgung sowohl für Mediumzufluß, Sauerstoffversorgung und Meiumabfluß gewährleistet ist.

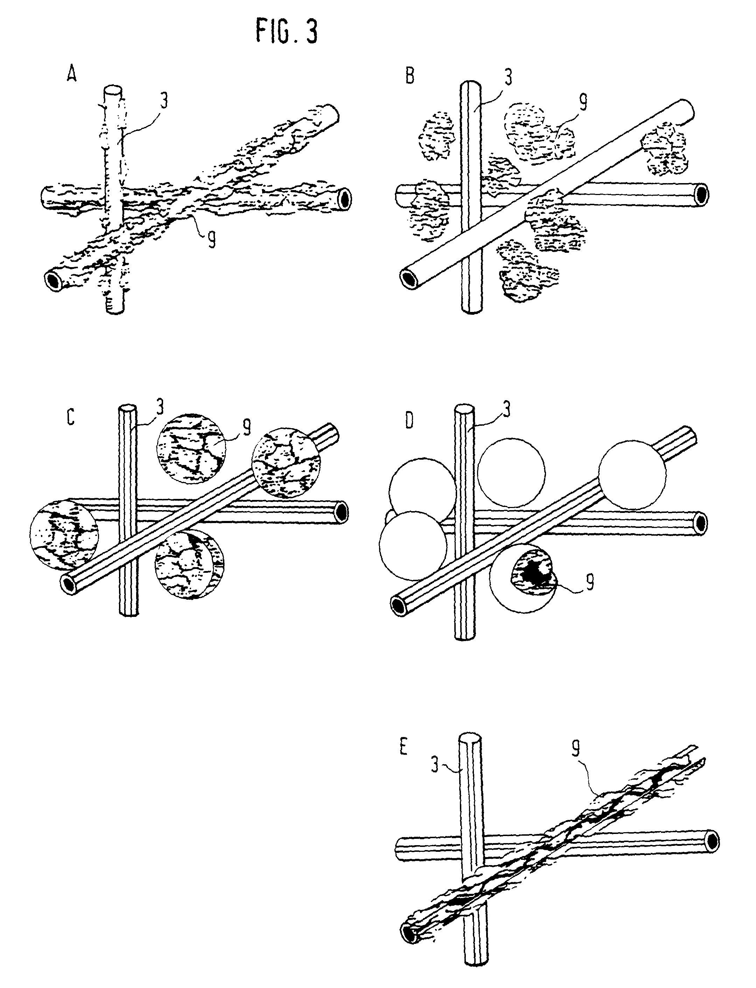

Fig. 2 zeigt jeweils einzelne Hohlfasern 3 von drei unabhängigen Hohlfasermembransystemen. Fig. 3a und b zeigen dabei eine beliebig herausgegriffene Stelle aus dem Innenbereich 4 des Moduls 1. Aus Fig. 2a und b ist der wesentliche Erfindungsgedanke erkennbar. Durch die Hohlfasermembran 3, die ein einzelnes Membran aus einem ersten unabhängigen Membransystem darstellt, wird Medium zugeführt (durch Pfeile angedeutet). Dieses Medium tritt durch die Membran 3 hindurch. Die Mikroorganismen 9, hier Zellen, befinden sich dabei in den Hohlräumen des dicht gepackten Netzwerkes 5 und/oder sind, wie in Fig. 3 gezeigt, an den Kapillaren adhäriert. Zwischen den Zellen 9 und dem Medium findet dann ein Stoffaustausch statt. Erfindungsgemäß wird dabei durch ein weiteres unabhängiges Hohlfasermembransystem, hier in Fig. 2a und 2b herausgegriffen eine einzelne Hohlfasermembran 3, die Zellen z.B. mit Sauerstoff versorgt (horizontale Membrane). Ein drittes unabhängiges Hohlfasermembransystem, hier wiederum nur durch eine einzelne Hohlfasermembran 3 dargestellt, dient zum Mediumausstrom. Nach dem Stoffaustausch des Mediums mit den Zellen wird demgemäß das Medium abgeführt.

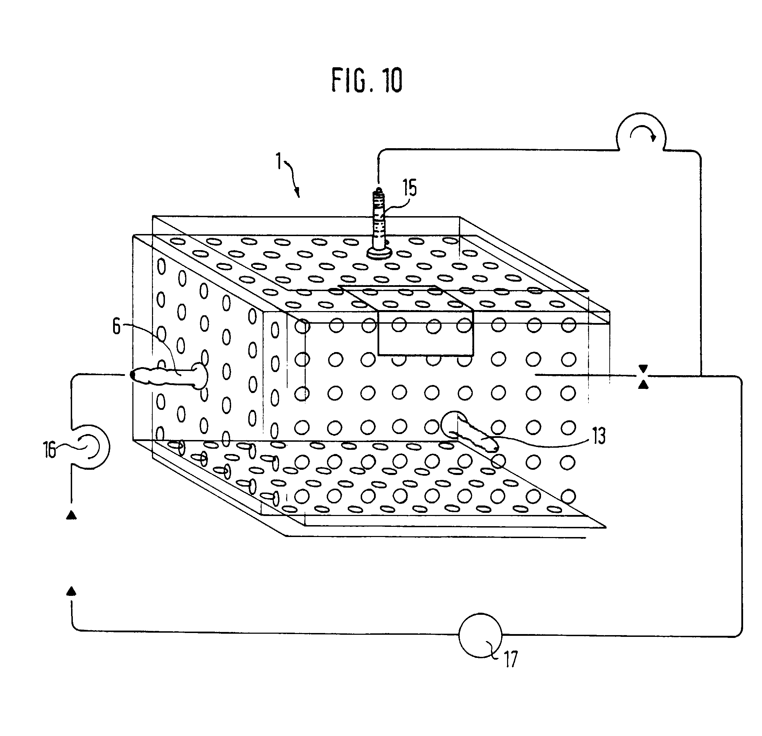

Die in den Fig. 2a und 2b abgebildeten einzelnen Hohlfasermembranen sind Bestandteile des dicht gepackten Netzwerkes 5 im Inneren des Moduls 1. Das dicht gepackte Netzwerk 5 ist dabei so aufgebaut (Fig. 1, 10), daß an jeder Stelle, innerhalb des Moduls 1, gleiche Bedingungen der Substratversorgung vorherrschen. An jeder Stelle des Moduls 1 werden die Zellen 9 demnach gleichmäßig versorgt und damit wird ein optimaler Stoffaustausch und eine Steuerbarkeit des Stoffaustausches gewährleistet. Aus Fig. 2b ist die Perfusion der Zellen 9 und der Mediumstrom entlang der Kapillare ersichtlich. Durch diese erfindungsgemäße Ausgestaltung kommt man nun den wahren Verhältnissen, z.B. in der Leber sehr nahe.

Fig. 3 zeigt verschiedene Zellkulturmethoden innerhalb des Moduls. Erfindungsgemäß können die Mikroorganismen 9 auf verschiedene Weise im Modul 1 angeordnet sind. Fig. 3a zeigt eine Adhäsionskultur an Membranen, Fig. 3b eine Aggregatkultur zwischen den Membranen, Fig. 3c Mikrokarrierkulturen zwischen den Membranen, Fig. 3d Kapselkulturen zwischen den Membranen und Fig. 3e Kokulturen in separaten Membrankompartments. Durch den erfindungsgemäßen Aufbau des Moduls 1, d.h. durch das dicht gepackte Netzwerk im Innenbereich des Moduls bestehend aus einzelnen Hohlfasermembranen, lassen sich somit verschiedene Zellkulturmethoden anwenden und damit eine große Anwendungsbreite für die Erfindung realisieren.

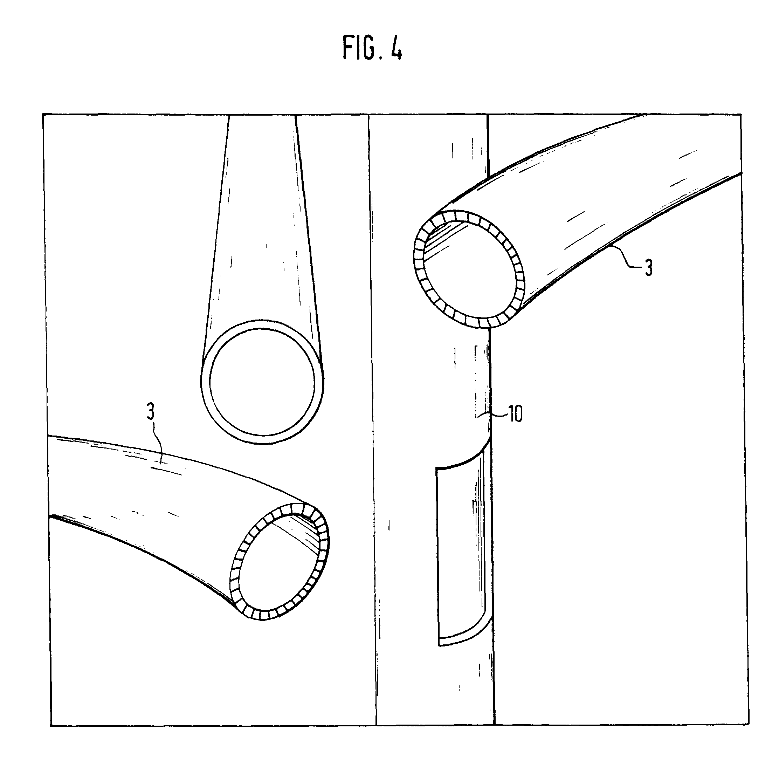

Fig. 4 zeigt nun jeweils einzelne Kapillare von vier unabhängigen Systemen und wie die Kapillaren beschichtet sind mit Adhäsionsfaktoren. Drei Systeme liegen dabei in einer Ebene. Fig. 4 zeigt herausgegriffen vier unabhängige Membransysteme, die jeweils Bestandteil eines gesamten Systems sind. Ein Hohlfasermembransystem 3 dient dabei, wie bereits in Fig. 1 und 2 beschrieben, zum Mediumeinfluß, ein System 3 zum Medienausfluß, ein drittes System zur Oxygenierung und ein weiteres unabhängiges System 10 z.B. zur Dialyse, als Wärmeaustauscher für Tiefgefrierung von Zellen oder für Kokulturen mit weiteren Zelltypen. Die schwarzen Stellen an den Membranen 3 zeigen die Porenöffnungen (Zellen nicht abgebildet). Durch die Bereitstellung von vier verschiedenen unabhängigen Hohlfasersystemen läßt sich somit der Anwendungsbereich des Erfindungsgegenstandes nochmals deutlich erweitern. Neben dem wesentlichen Vorteil der Erfindung, daß eine gleiche Substratversorgung für nahezu jede Zelle innerhalb des Moduls vorliegt, ermöglicht es die Erfindung auch, durch die Beschichtung der Kapillaren mit Adhäsionsfaktoren innerhalb des Moduls eine möglichst große Menge an Zellen unterzubringen, die nahezu alle eine gleiche Substratversorgung erfahren, und deshalb eine erhöhte Lebensdauer aufweisen.

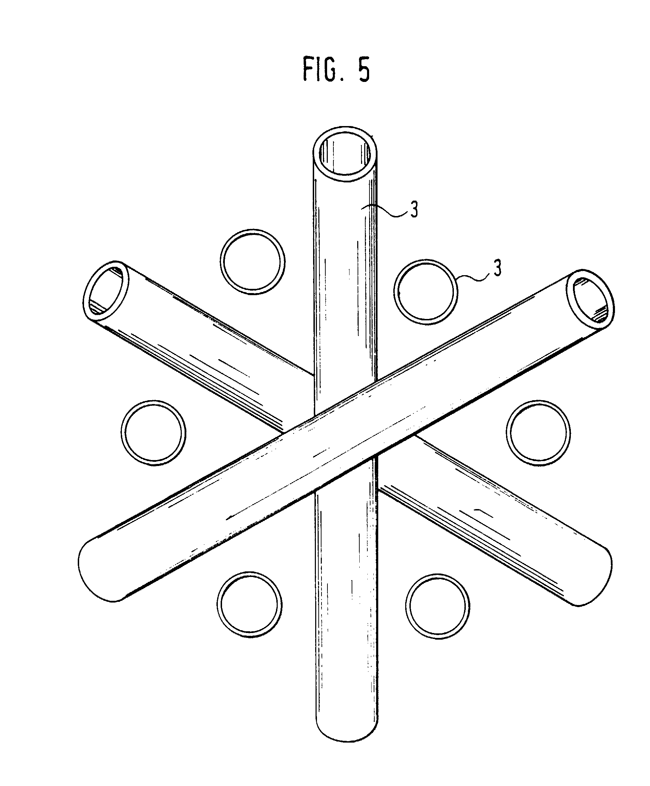

Fig. 5 zeigt nun in einer Draufsicht, in dreidimensionaler Ausgestaltung, eine Ebene eines Moduls mit vier unabhängigen Hohlfasermembransystemen, analog Fig. 4. Ein erstes unabhängiges System dient hierzu wieder zum Mediumeinstrom, ein zweites zum Mediumausstrom und ein drittes zur Sauerstoffversorgung. Ein viertes unabhängiges Hohlfasersystem kann zur Dialyse oder als Wärmeaustauscher für Tiefgefrierung der Zellen oder auch für Kokulturen dienen.

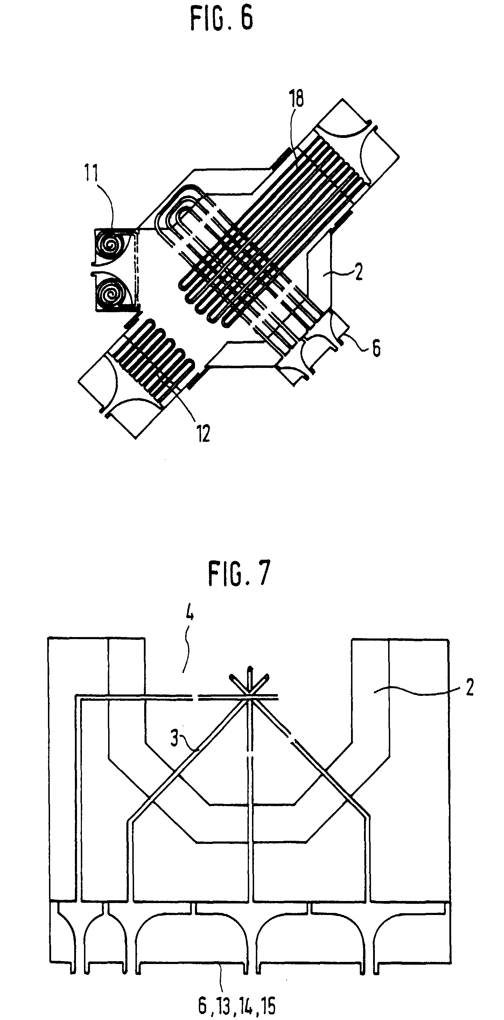

Fig. 6 zeigt verschiedene Möglichkeiten des Mediumausflusses aus dem Modul 1. In den bisher in den Fig. 1 bis 5 beschriebenen Ausgestaltungen der Erfindung erfolgte der Mediumausfluß jeweils über Hohlfasermembrane 3. Eine erfindungsgemäße Ausgestaltung sieht vor, daß der Mediumausfluß nihct nur durch Hohlfasermembrane, die Bestandteil eines unabhängigen Hohlfasermembransystems innerhalb des dicht gepackten Netzwerkes 5 sind, erfolgt, sondern daß der Mediumausfluß noch durch verschiedene Varianten erfolgen kann. Fig. 6 zeigt dabei in einem Bild die verschiedenen Möglichkeiten. Entweder erfolgt der Ausfluß über fest eingefügte Kapillarmembrane (verbunden mit dem Auslaßkopf 6), räumlich in vorgegebener Anordnung zu den übrigen Kapillarmembranen oder über auswechselbare Kapillarmembrane 12, ebenfalls räumlich unabhängig zu den übrigen Membransystemen. Die auswechselbare Kapillarmembran 12 ist dabei am Außengehäuse 2 befestigt. Das Außengehäuse 2 muß dabei eine Öffnung aufweisen, die dazu geeignet ist, die auswechselbare Kapillarmembran 12 aufzunehmen.

Eine weitere Variante besteht darin, am Außengehäuse 2 ebenfalls auswechselbare Flachmembrane 11 anzuordnen. Hierzu muß das Außengehäuse 2 wiederum entsprechende Öffnungen aufweisen.

Eine weitere Variante sieht austauschbare Kapillarmembrane 18 vor. Diese können entgegen denen, die mit dem Ausgang 6 verbunden sind, bei Verstopfung herausgezogen werden.

Entscheidend ist hierbei, daß die jeweiligen speziellen Mediumausflußvorrichtungen unabhängig zu den weiteren Hohlfasermembransystemen sind. Diese verschiedenen Mediumausflußvorrichtungen können auch zusätzlich zu einem bereits vorhandenen Mediumausfluß über eine Hohlfaserkapillarmembran, die Bestandteil des dicht gepackten Netzwerkes ist, erfolgen.

Fig. 7 zeigt eine spezielle Anordnung von Membranköpfen 6, 13, 14, 15 auf einer einzigen Seite des Moduls 1. Diese Ausgestaltung der Erfindung, d.h. die Anordnung aller Zu- und Abflüsse an den Außenflächen des Moduls 1 auf einer einzigen Seite, hat den Vorteil, daß hierbei alle Anschlüsse zu einer Ebene geführt werden können.

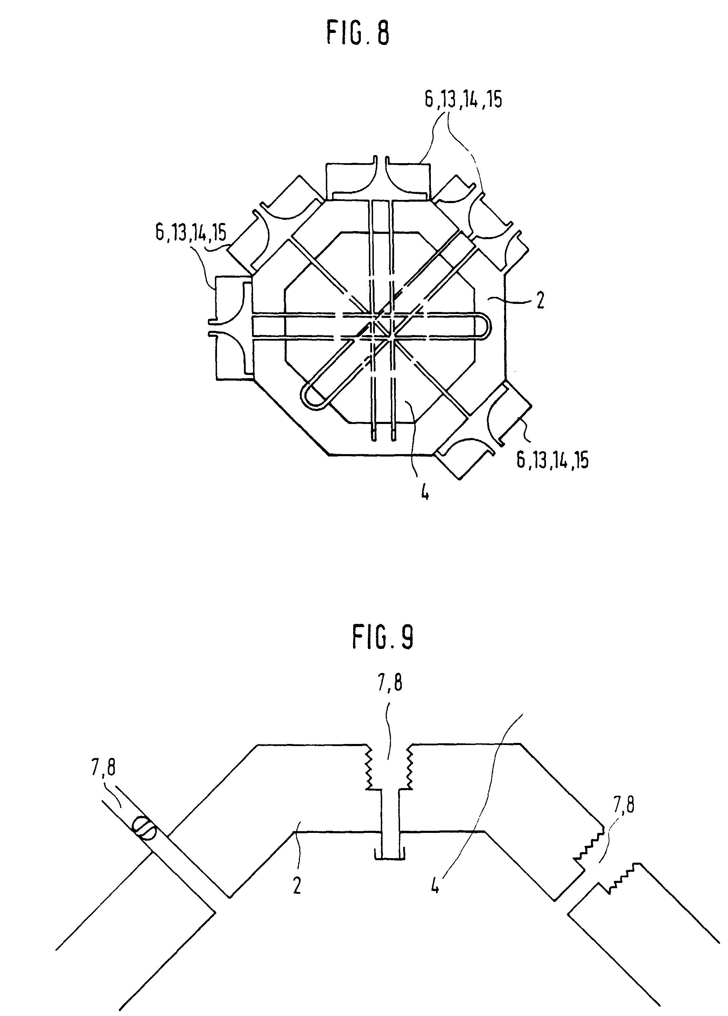

Fig. 8 zeigt eine Querschnittszeichnung eines achteckigen Moduls mit Kapillarmembranen und Membranköpfen 6, 13, 14, 15. Fig. 8 zeigt wiederum die verschiedenen Möglichkeiten auf, wie die Kapillare in einer Ebene geführt werden können. So ist es möglich, die Kapillare einfach doppeläufig zu führen mit einem Kopf für Mediumein- und ausfluß. Eine weitere Variante ist eine Kapillare einfach gerade mit einem entsprechenden Kopf für den Mediumeinfluß. Eine dritte Möglichkeit besteht darin, eine Kapillare einfach gerade mit totem Ende zu verwenden und einen Kopf für Mediumein- und ausfluß. Eine vierte Möglichkeit besteht in einer doppeläufigen Kapillare mit Zirkulation und einen Kopf für Mediumein- und -ausfluß.

Fig. 9 zeigt verschiedene Zugänge zum Innenbereich 4 des Moduls. Das Modul kann demnach verschiedene Zugänge 7, 8 aufweisen, um Messungen für Druck und Temperatur sowie Fluß, pH oder Osmolarität vorzunehmen.

Fig. 10 zeigt nun die Verwendung eines Moduls 1 als extrakorporales Leberunterstützungssystem. Das Medium, hier das Blutplasma, wird dabei über eine Blutpumpe 16 in dem Mediumeinstromkopf 6 des Moduls zugeführt. Die Sauerstoffversorgung erfolgt über den Einstromkopf 13. Über den Ausstromkopf 14 wird der Sauerstoff wieder abgeführt (nicht abgebildet). Die Mediumabfuhr erfolgt über den Mediumausstromkopf 15 und entsprechende Kontroll- und Pumpeinheiten 17.

Alle Zellen im System haben durch die erfindungsgemäße Ausgestaltung gleiche Bedingungen der Substratversorgung. Das zuführende Medium wird an alle Orte im Modul gleichförmig geführt, ebenso die Sauerstoffzufuhr. Durch Verwendung verschiedener ausführender Kapillare ist eine selektive Entnahme z.B. liprophiler Substrate möglich.

Durch diese erfindungsgemäße Ausgestaltung konnte die Lebensdauer (z.B. von Leberzellen) von 10 Tagen auf 7 Wochen gesteigert werden und die Aktivität um 40 % erhöht werden.

## Patentansprüche

1. Modul (1) zur Züchtung und zur Nutzung der Stoffwechselleistung und/oder zum Erhalt von Mikroorganismen (9), insbesondere für Zellen oder Bakterien, bestehend aus einem Außengehäuse (2), mindestens drei unabhängigen Membransystemen, die als Hohlfasermembrane (3) ausgebildet und im Innenbereich (4) des Moduls (1) angeordnet sind, und daß diese Hohlfasermembrane (3) ein dicht gepacktes räumliches Netzwerk (5) bilden und Mikroorganismen (9), die sich in den Hohlräumen des Netzwerkes (5) befinden und/oder an den Hohlfasermembranen (3) adhäriert sind, wobei das Netzwerk (5) aus sich kreuzenden und/oder überlagernden Hohlfasermembranen (3) besteht und so aufgebaut ist, daß die Mikroorganismen (9) an jeder Stelle im Innenbereich (4) des Moduls (1) nahezu gleiche Bedingungen der Substratversorgung und -entsorgung haben, wobei das dicht gepackte Netzwerk (5) zusätzlich ein weiteres flüssigkeitsimpermeables unabhängiges Kapillarsystem aufweist.

2. Modul nach Anspruch 1, dadurch gekennzeichnet, daß zusätzlich am Außengehäuse (2) eine auswechselbare Flachmembrane (11) oder Kapillarmembrane (12) angeordnet ist.

3. Modul nach Anspruch 1 oder 2, dadurch gekennzeichnet, daß das dicht gepackte Netzwerk (5) zusätzlich ein weiteres unabhängiges Kapillarsystem für Dialysezwecke aufweist.

4. Modul nach Anspruch 1 bis 3, dadurch gekennzeichnet, daß das Außengehäuse (2) durch eine Ausgußmasse gebildet wird, wobei ein Zugang von außen in das Lumen der Kapillaren oder Hohlfasermembranen ermöglicht ist.

5. Modul nach Anspruch 1 bis 4, dadurch gekennzeichnet, daß für den Ein- und/oder Auslaß, in das Lumen der Kapillaren oder Hohlfasermembranen entsprechende Ein- und/oder Auslaßköpfe (6, 13, 14, 15) vorgesehen sind, die mit den jeweiligen unabhängigen Kapillarsystemen kommunizieren.

6. Modul nach Anspruch 1 bis 5, dadurch gekennzeichnet, daß im Außengehäuse (2) des Moduls ein oder mehrere Zugänge (7, 8) vorgesehen sind, die in den Innenbereich (4) führen, um Mikroorganismen (9) in das Modul (1) einzufüllen und/oder Druck, Temperatur und/oder pH-Messungen durchzuführen.

7. Modul nach Anspruch 6, dadurch gekennzeichnet, daß die Zugänge (7) sich als perforierte Röhren in das Modul hinein fortsetzen, wodurch eine gleichmäßige Verteilung der Mikroorganinsmen (9) in den Innenbereich (4) ermöglicht wird.

8. Verfahren zum Betreiben des Moduls nach den Ansprüchen 1 bis 7 zum Erhalt und zur Nutzung der Stoffwechselleistung und/oder Züchtung von Mikroorganismen, dadurch gekennzeichnet, daß der Innenbereich (4) des Moduls (1) mit Mikroorganismen (9) gefüllt und die für die Züchtung und/oder Erhalt nötigen Bedingungen eingestellt werden und daß über ein erstes unabhängiges Hohlfasermembransystem Medium zugeführt und daß die Mikroorganismen (9) über ein zweites unabhängiges Hohlfasermembransystem zusätzlich z.B. mit Sauerstoff versorgt und von CO₂ entsorgt werden und daß nach erfolgtem Stoffaustausch das Medium über ein drittes unabhängiges Membransystem abgeführt wird.

9. Verfahren nach Anspruch 8, dadurch gekennzeichnet, daß das dritte unabhängige Membransystem für den Mediumabfluß ein Hohlfasermembransystem ist.

10. Verfahren nach Anspruch 8 oder 9, dadurch gekennzeichnet, daß der Mediumausfluß zusätzlich am Außengehäuse (2) des Moduls (1) über eine auswechselbare Kapillarmembrane (12) oder Flachmembrane (11) erfolgt.

11. Verfahren nach Anspruch 8 bis 10, dadurch gekennzeichnet, daß die Mikroorganismen (9) sich in den Hohlräumen des Netzwerkes (5) befinden und/oder untereinander aggregieren und die Aggregate/Einzelzellen an die Kapillarwände adhärieren.

12. Verfahren nach mindestens einem der Ansprüche 8 bis 11, dadurch gekennzeichnet, daß eines oder mehrere der unabhängigen Kapillarsysteme zeitweilig für das Abkühlen des Modules (1) mit den Mikroorganismen (9) verwendet werden, indem diese mit einem Kühlmedium durchströmt werden.

13. Verfahren nach Anspruch 12, dadurch gekennzeichnet, daß das Modul (1) mit den enthaltenen Mikroorganismen (9) auf Temperaturen unter -20 °C abgekühlt wird, um die Mikroorganismen (9) über längere Zeiträume zu lagern bzw. zu transportieren.

14. Verfahren nach Anspruch 12 oder 13, dadurch gekennzeichnet, daß das Abkühlen des Moduls (1) mit den enthaltenden Mikroorganismen (9) nach dem Austausch des Zellnährungsmediums mit einem geeigneten Zellgefriermedium erfolgt.

15. Verfahren nach Anspruch 8 bis 14, dadurch gekennzeichnet, daß die Mikroorganismen Zellen, insbesondere Leberzellen sind.

16. Verwendung eines Moduls nach den Ansprüchen 1 bis 7 als Leberunterstützungssystem.

17. Verwendung eines Moduls nach den Ansprüchen 1 bis 7 als Bioreaktor alternativ zu Tierversuchen.

18. Verwendung eines Moduls nach den Ansprüchen 1 bis 7 zur Herstellung von biologischen Zellprodukten.

## Claims

1. Module (1) for growing and for exploiting the metabolic performance and/or for obtaining microorganisms (9), in particular for cells or bacteria, composed of an external housing (2), at least three independent membrane systems which are designed as hollow-fibre membranes (3) and arranged in the interior (4) of the module (1), these hollow-fibre membranes (3) forming a densely packed spatial reticulate structure (5) and microorganisms (9), which are located in the interstices of the reticulate structure (5) and/or adhere to the hollow-fibre membranes (3), in which the reticulate structure (5) is composed of intersecting and/or superimposed hollow-fibre membranes (3) and is constructed in such a way that the substrate supply and substrate disposal conditions for the microorganisms (9) are virtually identical at any given place in the interior (4) of the module (1), in which the densely packed reticulate structure (5) is additionally equipped with a further, liquid-impermeable independent capillary system.

2. Module according to Claim 1, characterized in that an exchangeable flat membrane (11) or capillary membrane (12) is additionally arranged on the external housing (2).

3. Module according to Claim 1 or 2, characterized in that the densely packed reticulate structure (5) is additionally equipped with a further independent capillary system for dialysis purposes.

4. Module according to Claims 1 to 3, characterized in that the external housing (2) is made of a filling composition, access from the outside to the lumen of the capillaries or of the hollow-fibre membranes being made possible.

5. Module according to Claims 1 to 4, characterized in that the intake and/or outlet of the lumen of the capillaries or hollow-fibre membranes are provided with suitable intake and/or outlet heads (6, 13, 14, 15) which communicate with the respective independent capillary systems.

6. Module according to Claims 1 to 5, characterized in that the external housing (2) of the module provides one or more accesses (7, 8) which lead into the interior (4), in order to charge the module (1) with microorganisms (9) and/or to carry out pressure, temperature and/or pH measurements.

7. Module according to Claim 6, characterized in that the accesses (7, 8) continue into the module in the form of perforated tubes, allowing the microorganisms (9) to be distributed uniformly in the interior (4).

8. Process for operating the module according to Claims 1 to 7 for obtaining or exploiting the metabolic performance and/or growing microorganisms, characterized in that the interior (4) of the module (1) is filled with microorganisms and the conditions required for growing and/or obtaining are set and in that medium is fed in via a first independent hollow-fibre membrane system and in that the microorganisms (9) are additionally provided with, for example, oxygen via a second independent hollow-fibre membrane system and their CO₂ is disposed of and in that, when the exchange of substances is complete, the medium is discharged via a third independent membrane system.

9. Process according to Claim 8, characterized in that the third independent membrane system for the medium discharge is a hollow-fibre membrane system.

10. Process according to Claim 8 or 9, characterized in that the medium discharge additionally takes place on the external housing (2) of the module (1) via an exchangeable capillary membrane (12) or flat membrane (11).

11. Process according to Claim 8 to 10, characterized in that the microorganisms (9) are located in the interstices of the reticulate structure (5) and/or aggregate with each other and the aggregates/single cells adhere to the capillary walls.

12. Process according to at least one of Claims 8 to 11, characterized in that one or more of the independent capillary systems are temporarily used for cooling the module (1) with the microorganisms (9) by allowing a cooling medium to flow through them.

13. Process according to Claim 12, characterized in that the module (1) with the microorganisms (9) which it contains is cooled to temperatures of below -20°C in order to transport or store the microorganisms (9) over prolonged periods.

14. Process according to Claim 12 or 13, characterized in that the module (1) with the microorganisms (9) which it contains is cooled with a suitable cell cryomedium after the cell nutrient medium has been exchanged.

15. Process according to Claims 8 to 14, characterized in that the microorganisms are cells, in particular hepatocytes.

16. Use of a module according to Claims 1 to 7 as liver support system.

17. Use of a module according to Claims 1 to 7 as bioreactor as alternative for animal experiments.

18. Use of a module according to Claims 1 to 7 for producing biological cell products.

## Revendications

1. Module (1) pour la culture et pour l'utilisation du potentiel métabolique et/ou pour la subsistance de micro-organismes (9), notamment pour des cellules ou des bactéries, constitué par un boîtier extérieur (2), au moins trois systèmes indépendants de membranes, qui sont agencés sous la forme de membranes (3) formées de fibres creuses et sont disposés dans une zone intérieure (4) du module (1) et dans lequel ces membranes (3) formées de fibres creuses forment un réseau spatial entassé de façon dense (5), et des micro-organismes (9) qui sont situés dans les cavités du réseau (5) et/ou adhèrent aux membranes (3) formées de fibres creuses, et dans lequel le réseau (5) est formé de membranes (3) formées de fibres creuses, qui se croisent et/ou sont superposées, et est agencé de telle sorte que les micro-organismes (9) possèdent en n'importe quel emplacement de la partie intérieure (4) du module (1), presque les mêmes conditions d'alimentation en substrat et d'évacuation de substrat, et dans lequel le réseau entassé de façon dense (5) comporte en outre un autre système capillaire indépendant, imperméable aux liquides.

2. Module selon la revendication 1, caractérisé en ce qu'une membrane capillaire (12) ou une membrane plate interchangeable (11) est prévue en supplément sur le boîtier extérieur (2).

3. Module selon la revendication 1 ou 2, caractérisé en ce que le réseau entassé de façon dense (5) comporte en outre un autre système capillaire indépendant, utilisé pour la dialyse.

4. Module selon les revendications 1 à 3, caractérisé en ce que le boîtier extérieur (2) est formé par une masse coulée, un accès dans la lumière des capillaires ou des membranes formées de fibres creuses étant possible à partir de l'extérieur.

5. Module selon les revendications 1 à 4, caractérisé en ce que pour l'entrée et/ou la sortie il est prévu des têtes d'entrée et/ou de sortie correspondantes (6,13,14,15) dans la lumière des capillaires ou des membranes formées de fibres creuses, têtes qui communiquent avec les systèmes respectifs indépendants de capillaires.

6. Module selon les revendications 1 à 5, caractérisé en ce que dans le boîtier extérieur (2) du module il est prévu un ou plusieurs accès (7,8), qui aboutissent dans la partie intérieure (15), pour l'introduction de micro-organismes (9) dans le module (1) et/ou l'exécution de mesures de pression, de température et/ou du pH.

7. Module selon la revendication 6, caractérisé en ce que les accès (7) se prolongent dans le module sous la forme de tubes perforés, une répartition uniforme des micro-organismes (9) dans la partie intérieure (15) étant ainsi rendue possible.

8. Procédé pour faire fonctionner le module selon les revendications 1 à 7 pour la subsistance et pour l'utilisation du potentiel métabolique et/ou la croissance de micro-organismes, caractérisé en ce qu'on remplit la partie intérieure (4) du module (1) avec des micro-organismes (9) et qu'on règle les conditions nécessaires pour la croissance et/ou la subsistance, et qu'un milieu est introduit par l'intermédiaire d'un premier système indépendant de membranes formées de fibres creuses et qu'on alimente en outre par exemple en oxygène les micro-organismes (9) par l'intermédiaire d'un second système indépendant de membranes formées de fibres creuses et qu'on évacue le CO₂ par ce système et qu'une fois réalisé l'échange de substances, le milieu est évacué par l'intermédiaire d'un troisième système indépendant de membranes.

9. Procédé selon la revendication 8, caractérisé en ce que le troisième système indépendant de membranes pour l'évacuation du milieu est un système de membranes formées de fibres creuses.

10. Procédé selon la revendication 8 ou 9, caractérisé en ce que l'évacuation du milieu s'effectue en outre au niveau du boîtier extérieur (2) du module (1) par l'intermédiaire d'une membrane capillaire remplaçable (12) ou d'une membrane plate remplaçable (11).

11. Procédé selon l'une des revendications 8 à 10, caractérisé en ce que les micro-organismes (9) sont situés dans les cavités du réseau (5) et/ou s'agrègent entre eux et que les agrégats/les cellules individuelles adhèrent aux parois des capillaires.

12. Procédé selon au moins l'une des revendications 8 à 11, caractérisé en ce qu'un ou plusieurs des systèmes indépendants de capillaires sont utilisés par intervalles pour le refroidissement du module (1) contenant les micro-organismes (9) par le fait qu'on fait circuler dans ces systèmes un fluide de refroidissement.

13. Procédé selon la revendication 12, caractérisé en ce qu'on refroidit le module (1) et les micro-organismes (9) qu'il contient, à des températures inférieures à -20°C de manière à stocker ou transporter les micro-organismes (9) sur des intervalles de temps plus longs.

14. Procédé selon la revendication 12 ou 13, caractérisé en ce que le refroidissement du module (1) avec les micro-organismes (9) qu'il contient, s'effectue après le remplacement du milieu nourricier des cellules avec un milieu approprié de réfrigération des cellules.

15. Procédé selon les revendications 8 à 14, caractérisé en ce que les micro-organismes sont des cellules, notamment des cellules du foie.

16. Utilisation d'un module selon les revendications 1 à 7, en tant que système d'assistance du fonctionnement du foie.

17. Utilisation d'un module selon les revendications 1 à 7 en tant que bioréacteur, à la place d'essais effectués sur des animaux.

18. Utilisation d'un module selon les revendications 1 à 7 pour fabriquer des produits sous forme de cellules biologiques.
